# EUROPEAN PATENT APPLICATION

(11) **EP 2 808 319 A1**
(43) Date of publication of application: **03.12.2014**
(21) Application number: 14170226.6
(22) Date of filing: 28.05.2014
(51) Int. Cl.: C07C 215/54, A61K 9/00, A61K 47/48

(54) **3-[3-(Dimethylamino)-1-ethyl-2-methylpropyl]phenol resin complex**

(30) Priority: 31.05.2013 US 201361829858 P; 31.05.2013 US 201361829816 P
(71) Applicant: Arevipharma GmbH, 01445 Radebeul (DE)
(72) Inventor: Hartenhauer, Helge Dr., 01187 Dresden (DE); Limmert, Michael Dr., 01277 Dresden (DE)
(74) Representative: Prüfer & Partner GbR European Patent Attorneys

(57) **Abstract**

The present invention discloses a 3-[3-(dimethylamino)-1-ethyl-2-methylpropyl]phenol resin complex, preferably an amorphous 3-[3-(dimethylamino)-1-ethyl-2-methylpropyl]phenol resin complex formed by the reaction between a cation exchange resin and 3-[3-(dimethylamino)-1-ethyl-2-methylpropyl]phenol or a pharmaceutically acceptable acid addition salt thereof. It can exhibit an in-vitro release rate of 3-[3-(dimethylamino)-1-ethyl-2-methylpropyl]phenol from the 3-[3-(dimethylamino)-1-ethyl-2-methylpropyl]phenol resin complex which is in accordance with the requirements of Ph. Eur. on "conventional-release dosage forms".

## Description

### Field of the Invention

The present invention relates to a novel form of 3-[3-(dimethylamino)-1-ethyl-2-methylpropyl]phenol and a process for the production thereof.

### Background of the invention

3-[3-(dimethylamino)-1-ethyl-2-methylpropyl]phenol3-[3-(dimethylamino)-1-ethyl-2-methylpropyl]phenol, and its physiologically acceptable salts are described in EP0693475. 3-[3-(dimethylamino)-1-ethyl-2-methylpropyl]phenol is a centrally acting analgesic with a dual mode of action as an agonist of the µ-opioid receptor and as a norepinephrine reuptake inhibitor. 3-[3-(dimethylamino)-1-ethyl-2-methylpropyl]phenol is approved for the treatment of moderate to severe acute pain such as post operative pain, cancer pain etc. In such cases nausea and vomiting is a frequently associated problem.

3-[3-(dimethylamino)-1-ethyl-2-methylpropyl]phenol exists in various polymorphic forms, which, on top of that, may vary in stability: for example, 3-[3-(dimethylamino)-1-ethyl-2-methylpropyl]phenol hydrochloride crystalline "Form A" may convert to "Form B" upon heating (see WO2006/00441), and polymorphic Forms A, B und C of 3-[3-(dimethylamino)-1-ethyl-2-methylpropyl]phenol free base, as described in WO2009/071310, are non-uniform in their solubility profiles. An amorphous form of 3-[3-(dimethylamino)-1-ethyl-2-methylpropyl]phenol hydrochloride is described in WO2011138037 and US2010272815A1. Various acid addition salt forms of 3-[3-(dimethylamino)-1-ethyl-2-methylpropyl]phenol are also known, as provided in US2011071120A1.

Polymorphism is defined as the ability of a substance to exist as two or more solid phases that have different arrangements of the individual molecules in a crystal lattice or in an arbitrarily oriented or non-ordered solid structure. Different polymorphs may differ in their physical properties such as melting point, solubility, X-ray diffraction patterns, differential scanning thermograms and the like. Although these differences disappear upon dissolution of the solids, they can influence the pharmaceutically relevant properties of the solid form, such as shelf life, handling, release rate, and even safety as will be stated for the novel form as described herein.

Additionally, various pharmaceutical dosage forms of 3-[3-(dimethylamino)-1-ethyl-2-methylpropyl]phenol, and its physiologically acceptable salts are known from the prior art, as described for example in WO02/67651, WO03/035053, WO2006/002886, WO2007/128412, WO2007/128413, WO2008/110323, WO2009/067703, WO2009/092601, US2010-272815, WO2011138037 and WO2012119728.

Thus, there is still a need for a uniform, stable, and easily obtainable form of 3-[3-(dimethylamino)-1-ethyl-2-methylpropyl]phenol, which is the object of the present invention.

### Summary of the invention

To solve this object, the present invention provides a [3-(dimethylamino)-1-ethyl-2-methylpropyl]phenol resin complex, preferably an amorphous [3-(dimethylamino)-1-ethyl-2-methylpropyl]phenol resin complex, formed between 3-[3-(dimethylamino)-1-ethyl-2-methylpropyl]phenol or a pharmaceutically acceptable acid addition salt thereof and a cation exchange resin. The preferred new amorphous form is easily and reproducibly obtained and not prone to phase transition, as it is known for the so-called "Form B" of 3-[(1 R,2R)-3-(dimethylamino)-1-ethyl-2-methylpropyl]phenol hydrochloride.
Preferably, said cation exchange resin is of synthetic type, more preferably is selected from styrene polymers and copolymers of styrene and divinylbenzene, respectively sulfonated or carboxylated, and copolymers of acrylic or methacrylic acid and divinylbenzene.

The new 3-[3-(dimethylamino)-1-ethyl-2-methylpropyl]phenol resin complex of the present invention exhibits and combines useful properties and improved performances, efficacy and compliance, such as patient friendliness by better taste profile of 3-[3-(dimethylamino)-1-ethyl-2-methylpropyl]phenol (3-[(1R,2R)-3-(dimethylamino)-1-ethyl-2-methylpropyl]phenol is described as bitter tasting according to WO2013011477); and controllable [3-(dimethylamino)-1-ethyl-2-methylpropyl]phenol release profiles combined with content uniformity and thus useful bioavailability characteristics, which are independent from 3-[3-(dimethylamino)-1-ethyl-2-methylpropyl]phenol polymorphism. In particular, the new 3-[3-(dimethylamino)-1-ethyl-2-methylpropyl]phenol resin complex of the present invention provides for further advantages regarding suitable and controllable variable 3-[3-(dimethylamino)-1-ethyl-2-methylpropyl]phenol release profiles whilst avoiding disadvantages known to prior art, such as interconversion problems as associated with its respective crystalline forms. Surprisingly, the release rate of 3-[3-(dimethylamino)-1-ethyl-2-methylpropyl]phenol, preferably in its amorphous form, from the 3-[3-(dimethylamino)-1-ethyl-2-methylpropyl]phenol resin complex meets the criteria of a "Conventional-release dosage forms" according to the Pharm. Euro. Such, the 3-[3-(dimethylamino)-1-ethyl-2-methylpropyl]phenol resin complex according to the invention has an in-vitro release rate of 3-[3-(dimethylamino)-1-ethyl-2-methylpropyl]phenol from the 3-[3-(dimethylamino)-1-ethyl-2-methylpropyl]phenol resin complex measured using the Ph. Eur. Paddle method in a medium according to Ph. Eur. at pH value which is below 7 at 37°C, preferably below 4 at 37°C, more preferably between 1 and 2 at 37°C.

In addition, the new 3-[3-(dimethylamino)-1-ethyl-2-methylpropyl]phenol resin complex of the present invention can be useful in the pursuit of the prevention of drug abuse. Furthermore, the 3-[3-(dimethylamino)-1-ethyl-2-methylpropyl]phenol resin complex according to the present invention, when provided as a powder form, may have good flowability and thereby good processability to produce pharmaceutical formulations with further excipients.

### Definitions

- Load factor:: ratio between exchange positions loaded with agent and theoretical total exchange capacity, occasionally also termed "content" herein
- Uptake:: ratio between exchange positions loaded with agent and total amount of agent
- Loading:: mass ratio between agent cation loaded on the exchange resin and the calculated total mass of loaded exchange resin

### Description of drawings

- Figure 1:: powder X-ray diffraction pattern of amorphous 3-[3-(dimethylamino)-1-ethyl-2-methylpropyl]phenol Amberlite IRP69 cation exchange resin complex
- Figure 2:: powder X-ray diffraction pattern of amorphous 3-[3-(dimethylamino)-1-ethyl-2-methylpropyl]phenol Purolite C100MRNS cation exchange resin complex
- Figure 3:: powder X-ray diffraction pattern of amorphous 3-[3-(dimethylamino)-1-ethyl-2-methylpropyl]phenol Amberlite IRP88 cation exchange resin complex
- Figure 4:: powder X-ray diffraction pattern of amorphous 3-[3-(dimethylamino)-1-ethyl-2-methylpropyl]phenol Amberlite IRP64 cation exchange resin complex
- Figure 5:: reference powder X-ray diffraction pattern of crystalline 3-[3-(dimethylamino)-1-ethyl-2-methylpropyl]phenol hydrochloride "Form A"
- Figure 6:: reference powder X-ray diffraction pattern of crystalline 3-[3-(dimethylamino)-1-ethyl-2-methylpropyl]phenol "Form A" according to and adopted from EP1612203B1
- Figure 7:: loading kinetic of 3-[3-(dimethylamino)-1-ethyl-2-methylpropyl]phenol on Amberlite IRP69 cation exchange resin
- Figure 8:: loading kinetic of 3-[3-(dimethylamino)-1-ethyl-2-methylpropyl]phenol on Purolite C100MRNS cation exchange resin
- Figure 9:: loading kinetic of 3-[3-(dimethylamino)-1-ethyl-2-methylpropyl]phenol on Amberlite IRP88 cation exchange resin
- Figure 10:: loading kinetic of 3-[3-(dimethylamino)-1-ethyl-2-methylpropyl]phenol on Amberlite IRP88 cation exchange resin
- Figure 11:: loading kinetic of 3-[3-(dimethylamino)-1-ethyl-2-methylpropyl]phenol on Amberlite IRP64 cation exchange resin
- Figure 12:: release kinetic of of 3-[3-(dimethylamino)-1-ethyl-2-methylpropyl]phenol on Amberlite IRP69 cation exchange resin
- Figure 13:: release kinetic of 3-[3-(dimethylamino)-1-ethyl-2-methylpropyl]phenol on Purolite C100MRNS cation exchange resin
- Figure 14:: release kinetic of 3-[3-(dimethylamino)-1-ethyl-2-methylpropyl]phenol on Amberlite IRP88 cation exchange resin

### Detailed description of the invention

In one embodiment, the new form of 3-[3-(dimethylamino)-1-ethyl-2-methylpropyl]phenol comprises - or consists of - a complex formed by the reaction between 3-[3-(dimethylamino)-1-ethyl-2-methylpropyl]phenol and the cation exchange resin, which may be referred to as "3-[3-(dimethylamino)-1-ethyl-2-methylpropyl]phenol resin complex" occasionally in the description below. In the 3-[3-(dimethylamino)-1-ethyl-2-methylpropyl]phenol resin complex, 3-[3-(dimethylamino)-1-ethyl-2-methylpropyl]phenol is chemically bound to the anionic groups of the resin.

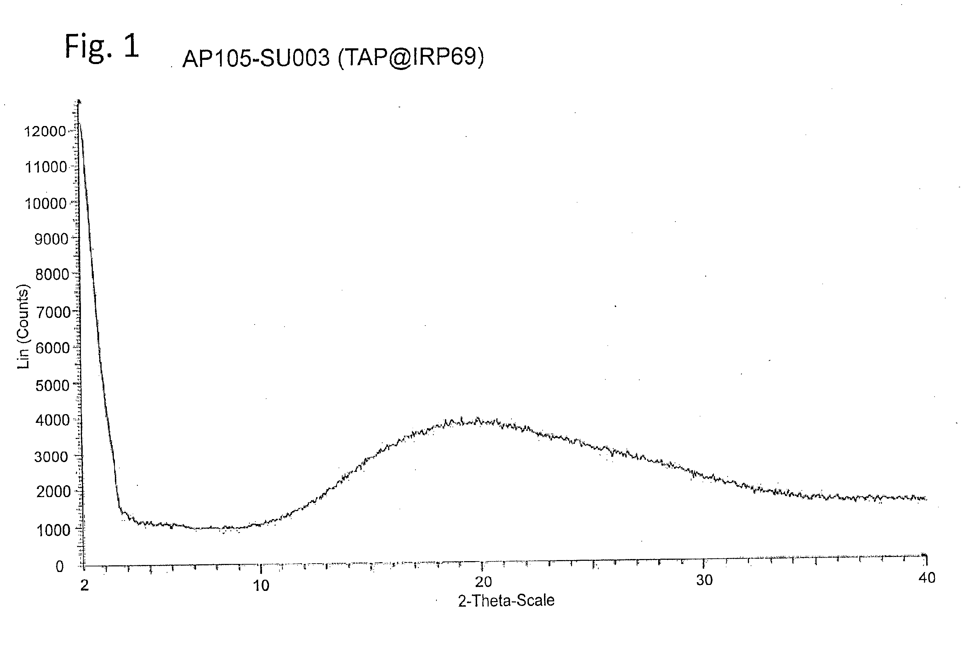

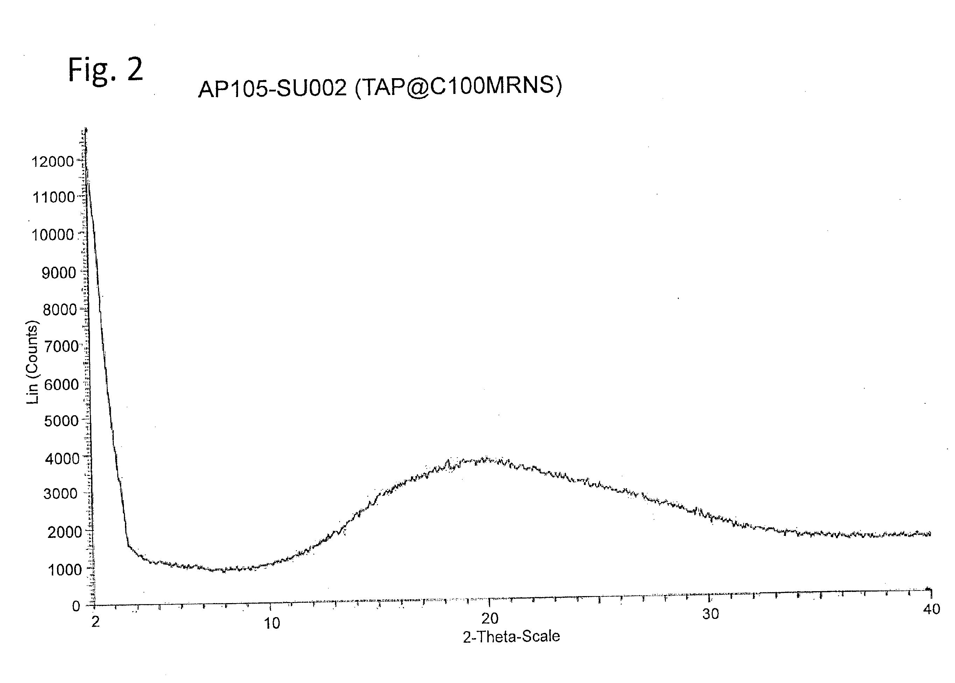

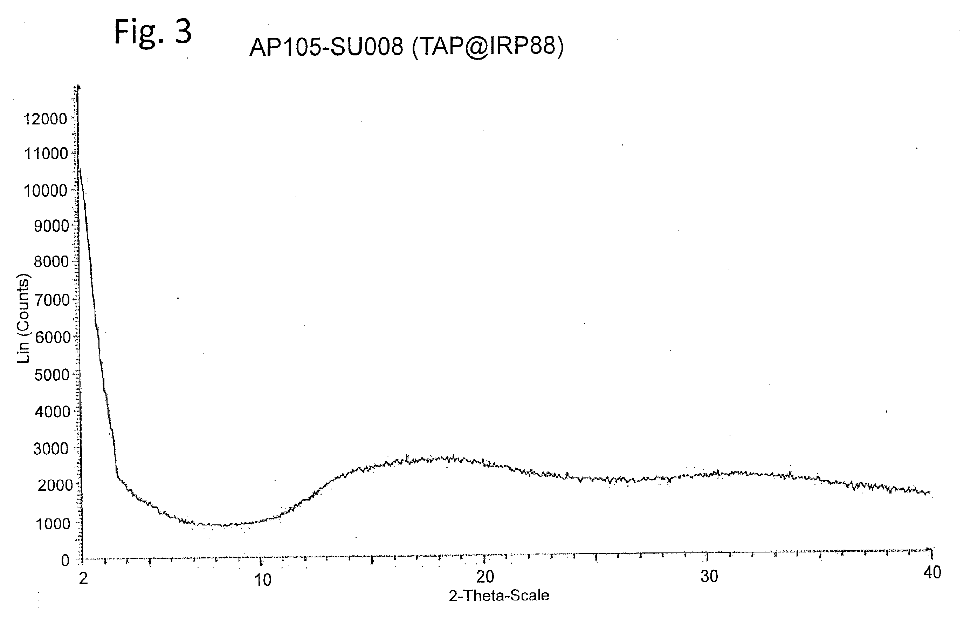

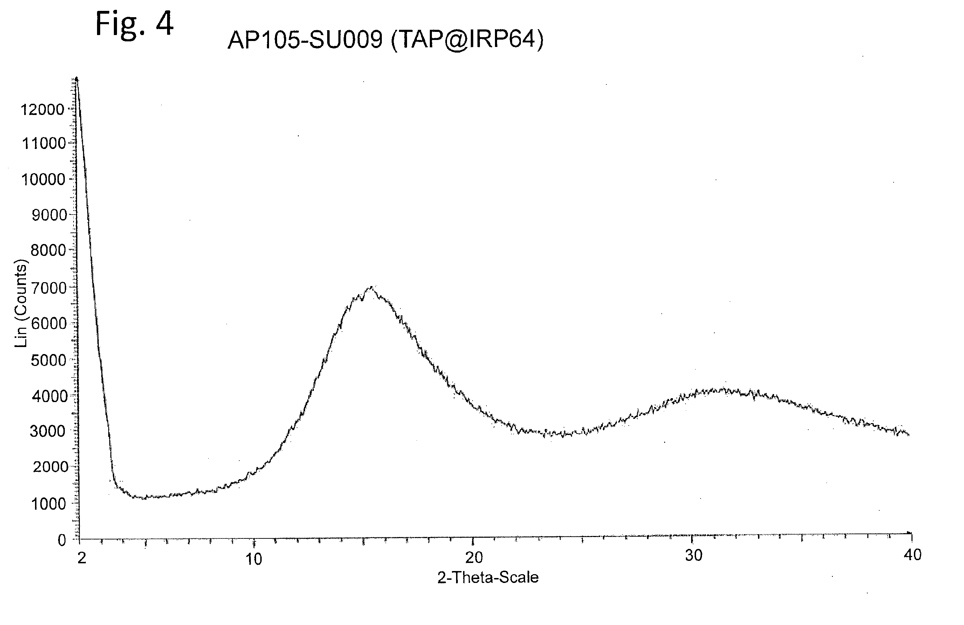

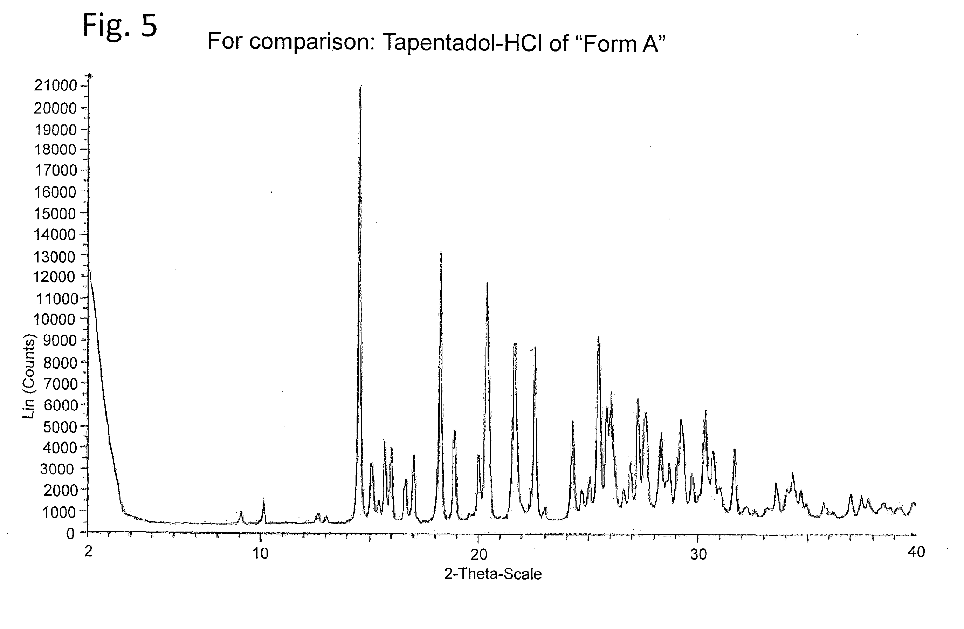

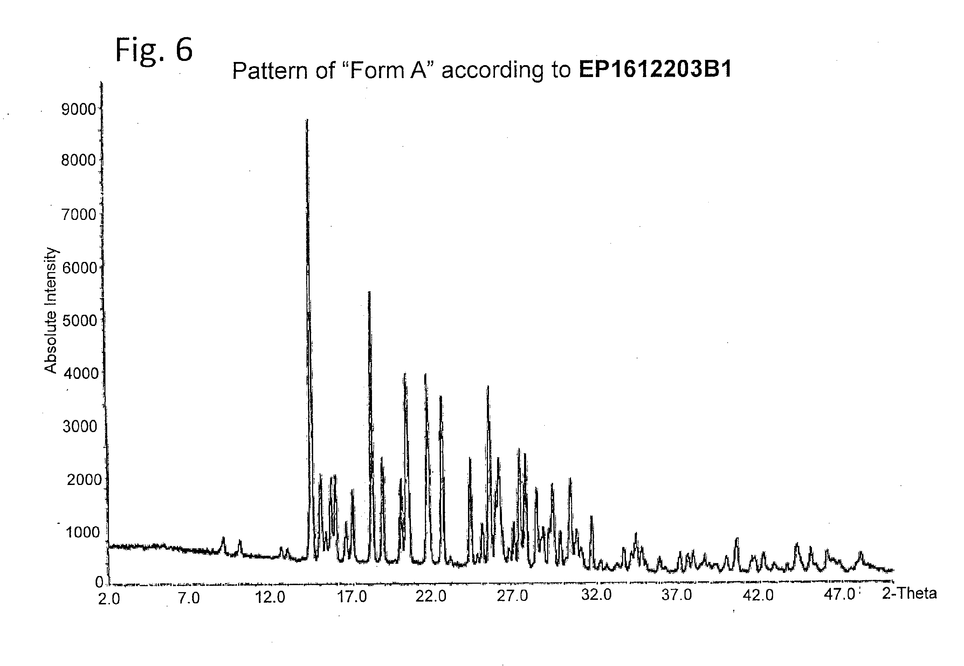

Preferably, the new form of 3-[3-(dimethylamino)-1-ethyl-2-methylpropyl]phenol is an amorphous form of 3-[(1R,2R)-3-(dimethylamino)-1-ethyl-2-methylpropyl]phenol comprising a complex formed by 3-[(1R,2R)-3-(dimethylamino)-1-ethyl-2-methylpropyl]phenol by a reaction with the cation exchange resin. Said complex formed is amorphous as well. The amorphous form of the 3-[(1R,2R)-3-(dimethylamino)-1-ethyl-2-methylpropyl]phenol resin complex is characterized by a powder X-ray diffraction pattern substantially void of discernable, discrete and sharp peaks. This can be seen and was confimed across various types and species of cation exchange resins, exemplified with e.g. Amberlite^{™} IRP69-, Purolite C100MRNS-, Amberlite^{™} IRP88-, and Amberlite^{™} IRP64 - 3-[(1R,2R)-3-(dimethylamino)-1-ethyl-2-methylpropyl]phenol complexes shown in Figs. 1-4 in comparison to the 3-[3-(dimethylamino)-1-ethyl-2-methylpropyl]phenol hydrochloride crystalline "Form A", for example according to EP1612203B1, shown in Figs. 5 and 6. Said diffractogram, e.g. according to Figs. 1-4, indicates the amorphous nature of the new form of 3-[(1 R,2R)-3-(dimethylamino)-1-ethyl-2-methylpropyl]phenol. The parent resin may either show a powder X-ray diffraction pattern substantially void of discernable, discrete and sharp peaks, or may feature a propensity to yield an amorphous form upon complexation, upon solvent intercalation or maceration and the like, which essentially leads to a break-up of accidently occuring crystalline domains in the polymer.

Suitable cationic exchange resins for the 3-[3-(dimethylamino)-1-ethyl-2-methylpropyl]phenol-resin complex of the present invention are selected from styrene polymers, polystyrene polymers and copolymers of styrene and divinylbenzene, respectively sulphonated or carboxylated, and copolymers of acrylic or methacrylic acid and divinylbenzene.
The cation exchange resin is suitably selected on the basis of the mentioned composing polymers and copolymers. Further selection criteria include for example loading capacities and loading behaviour for the compound 3-[3-(dimethylamino)-1-ethyl-2-methylpropyl]phenol; release performance of the compound from the respective resin as a function of pH; swelling properties of the resin in aqueous media, which may also influence disintegration in aqueous media; particle size and particle size distribution of the resin; etc.
For example, the particle size distribution of the chosen resin may exert an influence, because loading and release is mainly driven by diffusion processes of the compound and water into the resin and from the resin, respectively. A relatively narrow particle size distribution and fine overall dispersion allows for relatively shorter loading times and more reproducible contents of the compound in the resin. Preferably, the cation exchange resins as used herein are fine, free flowing powders which allow for advantageous pharmaceutical manufacture of the final dosage form.
As a further example, the larger the swelling capacity (e.g. Amberlite IRP88) the stronger is a tendency and speed of disintegration of a final pharmaceutical formulation.

Preferred cationic exchange resins are divinylbenzene styrene sulfonate copolymer, polystyrene sulphonates and methacrylic acid divinylbenzene polymer.
Examples of divinylbenzene styrene sulfonate copolymer are Amberlite^{™} IRP69 (CAS #: 55464-99-8) and DOWEX^{™} resins, and examples of methacrylic acid divinylbenzene polymer are Amberlite^{™} IRP64 (CAS # 80892-32-6; Prolacrilex) and Amberlite^{™} IRP88 (CAS # 39394-76-5; Prolacrilin potassium) and an example for polystyrene sulphonates is Purolite C100MRNS (CAS # 63182-08-1). Amberlite^{™} resins are produced by Rohm&Haas and DOWEX^{™} resins are produced by DOW Chemical Company. Amberlite^{™} and Purolite resins are preferred, Amberlite^{™} IRP69, IRP88 and Purolite C100MRNS are more preferred and Amberlite^{™} IRP69 is particularly preferred, respectively in terms of loading and release properties for 3-[3-(dimethylamino)-1-ethyl-2-methylpropyl]phenol.

The cation exchange resin as used for the 3-[3-(dimethylamino)-1-ethyl-2-methylpropyl]phenol resin complex of the present invention is characterized by individual ion exchange activity, such as capacity and rate; these can be attributed to the substrate polymer as well to the active exchange moieties: the cation exchange resin may be a copolymer of styrene or acrylic or methacrylic acid with a vinyl aromatic compound such as divinylbenzene; the resin may derive its exchange activity from either weakly or strongly acidic groups such as carboxylic acid or sulfonic acid groups, respectively.
Preferably, strength and stability of the 3-[3-(dimethylamino)-1-ethyl-2-methylpropyl]phenol resin complex and thus release rate of 3-[3-(dimethylamino)-1-ethyl-2-methylpropyl]phenol from the complex can be controlled by choosing strong or weak acid groups depending on the type of the cationic exchange resin/polymer, and can be adjusted further by pH or ionic strength of cations present in the use environment. Further embodiments which may adjust the release rate include: variation of the loading amount of 3-[3-(dimethylamino)-1-ethyl-2-methylpropyl]phenol; choice of a suitable particle size of the cationic exchange resin; application of a surface coating onto 3-[3-(dimethylamino)-1-ethyl-2-methylpropyl]phenol resin complex particles; and inclusion of rate-release controlling polymers into a further formulated pharmaceutical composition. Furthermore, it is possible to mix a 3-[3-(dimethylamino)-1-ethyl-2-methylpropyl]phenol resin complex with an unloaded cationic exchange resin, suitably at a ratio of 0.5:1 to 2:1 and preferably at a ratio of around 1:1 (e.g. 0.75:1 to 1.25:1), to thereby modify the shape and timing of the 3-[3-(dimethylamino)-1-ethyl-2-methylpropyl]phenol release profile and, optionally, obtain constant release rates.

For example, Amberlite^{™} IRP69 has strongly (sulfonic) acidic functional groups and uses sodium ions as exchange cations. Amberlite^{™} IRP88 and Amberlite^{™} IRP64 have weakly (carboxylic) acidic functional groups, the former using potassium and the latter hydrogen ions as exchange ions.

The sulfonic acid-derived or carboxylic acid group-derived anionic groups can be reacted with the acid addition salt of 3-[3-(dimethylamino)-1-ethyl-2-methylpropyl]phenol in order to form the desired 3-[3-(dimethylamino)-1-ethyl-2-methylpropyl]phenol resin complex. The cation exchange resin may be dispersible in water or an aqueous solution which typically applies to the respective 3-[3-(dimethylamino)-1-ethyl-2-methylpropyl]phenol resin complexes as well.

The preferred form of the cation exchange resin used to form the 3-[3-(dimethylamino)-1-ethyl-2-methylpropyl]phenol resin complex is as solid powder, which is normally insoluble however dispersible in the solvent or solvents chosen. Alternatively, another form of the cation exchange resin employed to form the 3-[3-(dimethylamino)-1-ethyl-2-methylpropyl]phenol resin complex has a limited molecular weight of the resin polymer, notably in a range of at least 5,000 and optionally up to about 5,000,000, suitably in the range from about 500,000 to about 1,000,000, whereby the obtained 3-[3-(dimethylamino)-1-ethyl-2-methylpropyl]phenol resin complex according to this alternative embodiment may be soluble in water or aqueous solutions.

In yet another alternative embodiment, the 3-[3-(dimethylamino)-1-ethyl-2-methylpropyl]phenol resin complex according to the present invention, individually or together with excipients, additives or vehicles as component of a formulation, may be melting in a use environment, e.g. upon administration. The preferred form however is 3-[3-(dimethylamino)-1-ethyl-2-methylpropyl]phenol resin complex as solid powdery form, which is insoluble but can be dispersed in solvents of choice such as water, aqueous solutions or in other use media.

The cation exchange resin prior to the reaction with 3-[3-(dimethylamino)-1-ethyl-2-methylpropyl]phenol may contain hydrogen ions (protons) or alkali metal ions e.g. as of sodium or potassium or alkaline earth metal cations e.g. such as calcium which are bound to the anionic groups. The resin may be pretreated with a strong acid in order to recover the full ionic binding capacity; it may then be washed with water or organic solvent and finally be used to form the complex with 3-[3-(dimethylamino)-1-ethyl-2-methylpropyl]phenol (free base or salt form) with the resin. After formation of the complex between 3-[3-(dimethylamino)-1-ethyl-2-methylpropyl]phenol and the cation exchange resin, the remaining, i.e. non 3-[3-(dimethylamino)-1-ethyl-2-methylpropyl]phenol complexed, anionic groups (sulfonic acid groups or carboxylic acid groups) may be masked by yet another suitable counter ion. For example it may be selected from the group of alkali metal ions, alkaline earth metal ions and amines or ammonium ions.
Of these optionally utilized, additional counter ions in the final 3-[3-(dimethylamino)-1-ethyl-2-methylpropyl]phenol resin complex may one or several be selected from a group comprising alkali metals such as sodium and/or potassium, alkaline earth metals such as calcium or magnesium, and amines such as ammonia, methylamine, ethylamine, ethanolamine, alginine and histidine.

The 3-[3-(dimethylamino)-1-ethyl-2-methylpropyl]phenol resin complex according to the present invention can be obtained in solid form. It can be applied in water or in aqueous media additionally containing one or more organic solvents. Examples of solvents may include, but are not limited to, one or more solvents comprising water, buffered aqueous solutions, acetone, methanol, ethanol, wherein water and buffered aqueous solutions are preferred. In case biphasic solutions are deemed advantageous, the list of organic solvents may further comprise diethyl ether, diisopropyl ether, t-butylmethyl ether and hexane.

The 3-[3-(dimethylamino)-1-ethyl-2-methylpropyl]phenol resin complex according to the present invention can be obtained by mixing 3-[3-(dimethylamino)-1-ethyl-2-methylpropyl]phenol (free base or acid adduct) and cation exchange resin in a weight ratio of 1:0.1 to 1:15, preferably 1:0.5 to 1:12, and more preferably 1:0.9 to 1:6 (calculated on the basis of 3-[3-(dimethylamino)-1-ethyl-2-methylpropyl]phenol free base).

Preferably, the 3-[3-(dimethylamino)-1-ethyl-2-methylpropyl]phenol resin complex obtained by the method described above is amorphous.

The 3-[3-(dimethylamino)-1-ethyl-2-methylpropyl]phenol content (or load factor) in the 3-[3-(dimethylamino)-1-ethyl-2-methylpropyl]phenol resin complex may vary over a wide range, for example 1% to 100%, but is preferably and beneficially adjusted in a specific range of 30% to 98%, more preferably 35% to 98%, even more preferably 40% to 98%, and in particular 45% to 95% of 3-[3-(dimethylamino)-1-ethyl-2-methylpropyl]phenol.

The term "3-[3-(dimethylamino)-1-ethyl-2-methylpropyl]phenol content in the 3-[3-(dimethylamino)-1-ethyl-2-methylpropyl]phenol resin complex" as used herein is defined as the ratio of the number of actually 3-[3-(dimethylamino)-1-ethyl-2-methylpropyl]phenol-occupied cationic exchange sites (sulfonate or carboxylate sites, for instance) to the total number theoretically available exchange sites of the resin (sulfonic or carboxylic acids, i.e. sites loaded with protons). For example when the theoretical capacity of a given amount of resin is calculated to be 10 mol and 8 mol of compound is calculated to be absorbed by the exchange resin the "3-[3-(dimethylamino)-1-ethyl-2-methylpropyl]phenol content in the 3-[3-(dimethylamino)-1-ethyl-2-methylpropyl]phenol resin complex" as defined herein is 80%. The number of theoretically available exchange sites is expressed as "capacity" of the ion exchange resin.

In a further embodiment, the invention provides a process for the preparation of 3-[3-(dimethylamino)-1-ethyl-2-methylpropyl]phenol resin complex which comprises stirring a cation exchange resin with 3-[3-(dimethylamino)-1-ethyl-2-methylpropyl]phenol or a physiologically acceptable acid addition salt thereof for a time sufficient to react and form the resin complex, in particular to form a complex between 3-[3-(dimethylamino)-1-ethyl-2-methylpropyl]phenol and the specified cation exchange resin in which the protons of the specified cation exchange resin are replaced by 3-[3-(dimethylamino)-1-ethyl-2-methylpropyl]phenol cations. The complex-forming reaction may be carried out in a medium such as water or a buffered aqueous solution, optinally containing an organic solvent such as methanol or ethanol. Preferably, water or a pH-adjusted aqueous solution, both optionally buffered, is used. For the complex-forming reaction, especially when using weak acidic cation exchange resin, preferably the pH of the complex-forming reaction solution is adjusted to a range from about pH 5 to about pH 10, more preferably to a range of pH 6 to 8. As a neutralizing agent, sodium hydroxide may be used.

The 3-[3-(dimethylamino)-1-ethyl-2-methylpropyl]phenol resin complex thus obtained can be further treated, for example, by rinsing with deionized water to remove any uncomplexed (e.g. free or surface-adsorbed) 3-[3-(dimethylamino)-1-ethyl-2-methylpropyl]phenol. Preferably, the isolated and dried powder composed of the 3-[3-(dimethylamino)-1-ethyl-2-methylpropyl]phenol resin complex contains substantially only 3-[3-(dimethylamino)-1-ethyl-2-methylpropyl]phenol resin complex, substantially free of remaining, non-reacted 3-[3-(dimethylamino)-1-ethyl-2-methylpropyl]phenol. Preferably, the 3-[3-(dimethylamino)-1-ethyl-2-methylpropyl]phenol resin complex thus obtained is amorphous.

In a further embodiment, the invention provides for the surprising effect of masking the taste of 3-[3-(dimethylamino)-1-ethyl-2-methylpropyl]phenol by the aforementioned method comprising stirring a cation exchange resin with 3-[3-(dimethylamino)-1-ethyl-2-methylpropyl]phenol or a physiologically acceptable acid addition salt thereof. The thus obtained 3-[3-(dimethylamino)-1-ethyl-2-methylpropyl]phenol resin complex is substantially free of the undesired, bitter taste as associated with 3-[3-(dimethylamino)-1-ethyl-2-methylpropyl]phenol. Preferably, the 3-[3-(dimethylamino)-1-ethyl-2-methylpropyl]phenol resin complex thus obtained is amorphous.

The 3-[3-(dimethylamino)-1-ethyl-2-methylpropyl]phenol resin complex according to the invention comprises 3-[3-(dimethylamino)-1-ethyl-2-methylpropyl]phenol or a pharmaceutically acceptable acid addition salt thereof, which contains or consist of 3-[(1R,2R)-3-(dimethylamino)-1-ethyl-2-methylpropyl]phenol or a pharmaceutically acceptable acid addition salt thereof or
3-[(1S,2S)-3-(dimethylamino)-1-ethyl-2-methylpropyl]phenol or a pharmaceutically acceptable acid addition salt thereof or
3-[(1R,2S)-3-(dimethylamino)-1-ethyl-2-methylpropyl]phenol or a pharmaceutically acceptable acid addition salt thereof or
3-[(1S,2R)-3-(dimethylamino)-1-ethyl-2-methylpropyl]phenol or a pharmaceutically acceptable acid addition salt thereof, wherein 3-[(1R,2R)-3-(dimethylamino)-1-ethyl-2-methylpropyl]phenol is preferred. Further, the 3-[3-(dimethylamino)-1-ethyl-2-methylpropyl]phenol or a pharmaceutically acceptable acid addition salt thereof according to the invention may contain any kind of mixture of the above mentioned stereoisomers, while the use of enantiomerically enriched or even enantio pure 3-[(1R,2R)-3-(dimethylamino)-1-ethyl-2-methylpropyl]phenol stereoisomer is again preferred.

The 3-[3-(dimethylamino)-1-ethyl-2-methylpropyl]phenol resin complex according to the present invention may be used in the manufacture of pharmaceutical compositions and may further be formulated into a variety of dosage forms, usually with additional pharmaceutically acceptable excipients, using common techniques such as blending, kneading, grinding, wet granulation, dry granulation, sieving, filling, compressing, lyophilization, spray-drying, fluid-bed drying, on their own or in combination. Suitable dosage forms may include capsules, tablets, films, effervescent tablets, chewable tablets, chewing gum, suspensions, sprinkle granules and powder for reconstitution in suspension, without being limited thereto. Preferably, the 3-[3-(dimethylamino)-1-ethyl-2-methylpropyl]phenol resin complex used in the manufacture of pharmaceutical compositions as described above is amorphous.

In a pharmaceutical composition, the amount of 3-[3-(dimethylamino)-1-ethyl-2-methylpropyl]phenol-resin complex according to the present invention may be in the range of 0.1 to 80wt.-%, preferably between 0.5 to 60wt.-%, and possibly between 1 to 30wt.-%, relative to the total weight of the pharmaceutical composition, which may further comprise various excipients in amounts required to a total of 100wt.-%.

Suitable pharmaceutically acceptable excipients, additives or vehicles can be selected from solvents or dispersing media, diluents, binders, disintegrants, stabilizers, fillers, buffers, coloring agents, sweetening agents, flavors, preservatives, lubricants, suspending agents, and mixtures thereof. Examples of such ingredients and their possible amounts, respectively in wt.-% relative to the total weight of the pharmaceutical composition, are provided as follows:
Suitable solvents, solvent combinations or dispersing media, of a content of 1 to 90% when present, may include solely or in combination water, aqueous solutions including buffered solutions and water-mixed media. Suitable one or more diluents, which when present may be contained at 1 to 30%, may include one or more selected from a group consisting of lactose, dextrose, microcrystalline cellulose and starch, without being limited thereto. Suitable one or more binders, which when present may be contained at 1 to 30%, may include polyvinyl pyrrolidone, hydroxypropyl cellulose, hydroxypropylmethyl cellulose, hydroxyethyl cellulose, sodium carboxymethyl cellulose, microcrystalline cellulose, dicalcium phosphate, gelatin, glycine, mannitol, hydrolyzed dextrose, dextran, dextrin, maltodextrin, alginates, pectin, carrageenan, agar, chitosan, sodium starch glycolate and sodium alginate, without being limited thereto. Suitable one or more disintegrants, which when present may be contained at 1 to 15 %, may include croscarmellose sodium, sodium starch glycolate, cross-linked polyvinyl pyrrolidone, starch, and low-substituted hydroxypropyl cellulose, without being limited thereto. Suitable one or more stabilizers, which when present may be contained at 0.01 to 5 %, may include polymers and nonionic and ionic surfactants, such as gelatin, glycerol monostearate, cetostearyl alcohol, sorbitan esters, polyoxyethylene alkyl ethers, polyoxyethylene sorbitan fatty acid esters (e.g. "Tween 20" or "Tween 80"), polyethylene glycol, polyvinylpyrrolidone, polyvinylalcohol, polyoxyethylene copolymers, polyoxypropylene copolymers, and polyethyleneoxide, without being limited thereto. Suitable one or more fillers, which when present may be contained at 1% to 60%, may include include calcium disulfate, calcium or magnesium carbonate, calcium phosphate, microcrystalline cellulose, lactose (in particular lactose monohydrate and lactose anhydrous), sucrose, mannitol, sorbitol, isomalt, glucose, maltose, dextrose, various starches and modified starches, without being limited thereto. Suitable one or more pH-value adjusting componds or buffers, which when present may be contained at 0.1 % to 20%, may comprise sodium hydroxide; boric, carbonic, phosphoric acid; acetic, succinic, malaic, tartaric, citric, benzoic, lactic, glyceric, gluconic, glutaric and glutamic acids; with respect to the aforementione acids their respective sodium, potassium and ammonium salts. Suitable one or more coloring agents, which when present may be contained at 0.1 to 10%, may include pigments such as iron oxide and titanium oxide, without being limited thereto. Suitable one or more sweetening agents, which when present may be contained at 0.1 to 10%, may include dextrose, sorbitol, mannitol, aspartame, acesulfame and citric acid, without being limited thereto. Suitable one or more preservatives, which when present may be contained at 0.1 to 5%, may include benzoic acid, methylparabene, ethylparabene and propylparabene, without being limited thereto. Suitable one or more lubricants, which when present may be contained at 0.1 to 10%, may include magnesium stearate, stearic acid, talcum, silica gel, colloidal silicon dioxide (such as Aerosil^{™} series) and sucrose fatty acid ester, without being limited thereto. Suitable one or more suspending agents, which when present may be contained at 0.1 to 10% and which serve for the 3-[3-(dimethylamino)-1-ethyl-2-methylpropyl]phenol resin complex to remain distributed in a suspension and thus maintain content uniformity of the active substance in suspension, may include propylene glycol, polyethylene glycol and glycerin, without being limited thereto.

A particular advantage of the use of the 3-[3-(dimethylamino)-1-ethyl-2-methylpropyl]phenol resin complex obtained according to the present invention is a suitable controlled release and dissolution characteristic of 3-[3-(dimethylamino)-1-ethyl-2-methylpropyl]phenol from the resin into the usable aqueous dissolution medium as described above, combined with an efficient taste masking effect which allows for absence or virtual absence of any other bitter taste masking agents. One may incorporate, or optionally one may omit, sweetening agents, flavoring agents and mixtures thereof into the formulation.
Suitable sweetening agents for example may include any natural or artificial sweetener such as glucose, dextrose, fructose, saccharin, cyclamate, aspartame, acesulfame-K, sucrose, sugar alcohols such as sorbitol, mannitol or xylitol, and mixtures thereof. Suitable flavoring agents, which may be present at 0-5% wt.%, may for example include synthetic flavor oils, flavoring aromatics, oils, and extracts derived from plants, leaves, flowers, fruits, stems and combinations thereof, such as spearmint, peppermint, lemon, orange, grape, apple, peach, strawberry, raspberry, cherry.

For example, the pharmaceutical composition according to the invention may comprise as excipients, relative to the total weight of the pharmaceutical composition:
1 to 30 wt.-% diluent(s); 1 to 30 wt.-% binder(s); 1 to 15 wt.-% disintegrant(s); 1% to 60 wt.-% filler(s); and optionally: 0.01 to 5 wt.-% stabilizer(s); 0.1 % to 20 wt.-% buffer(s); 0.1 to 10 wt.-% coloring agent(s); 0.1 to 10 wt.-% sweetening agent(s); 0 to 5% wt.-% flavoring agent(s) 0.1 to 5 wt.% preservative(s); 0.1 to 10 wt.% lubricant(s); and/or 0.1 to 10 wt.-% suspending agent(s).

As an alternative embodiment of the process for preparing a pharmaceutical composition using 3-[3-(dimethylamino)-l-ethyl-2-methylpropyl]phenol resin complex according to the present invention one may dissolve 3-[3-(dimethylamino)-1-ethyl-2-methylpropyl]phenol in an aqueous medium and add the cation exchange resin to form the 3-[3-(dimethylamino)-1-ethyl-2-methylpropyl]phenol resin complex in the presence of one or more excipients, wherein the excipient(s) may be selected from those as described above. Thereby the 3-[3-(dimethylamino)-1-ethyl-2-methylpropyl]phenol resin complex can be suspended directly into suitable vehicles.

Furthermore, the 3-[3-(dimethylamino)-1-ethyl-2-methylpropyl]phenol resin complex , optionally together with one or more exciptients or vehicles, may also be isolated and dried for later use, for example as powder. This dried, solid form may later be reconstituted for actual use, for example such as an oral liquid. Preferably, the 3-[3-(dimethylamino)-1-ethyl-2-methylpropyl]phenol resin complex used in the manufacture of pharmaceutical compositions as described above is amorphous.

### Examples

The invention will be illustrated in further detail with reference to examples which are merely illustrative and do not limit the scope of the invention.

### Example 1

120 g of Amberlite^{™} IRP69 are added to a solution of 3-[3-(dimethylamino)-1-ethyl-2-methylpropyl]phenol hydrochloride (30 g in 100 mL of water). The mixture is stirred at room temperature for about 4 hours. The resulting 3-[3-(dimethylamino)-1-ethyl-2-methylpropyl]phenol resin complex is filtered off and washed with water. The 3-[3-(dimethylamino)-1-ethyl-2-methylpropyl]phenol resin complex is then dried at 50 °C in vacuo.

### Example 2

125 g of Amberlite^{™} IRP64 are added to a solution of 3-[3-(dimethylamino)-1-ethyl-2-methylpropyl]phenol hydrochloride (25 g in 100 mL water). The pH of the solution is adjusted to about 7, and then the mixture is stirred at room temperature for about 4 hours. The resulting 3-[3-(dimethylamino)-1-ethyl-2-methylpropyl]phenol resin complex is filtered off and washed with water. The 3-[3-(dimethylamino)-1-ethyl-2-methylpropyl]phenol resin complex is then dried at 50 °C in vacuo.

### Example 3

180 g of Amberlite^{™} IRP88 are added to a solution of 3-[3-(dimethylamino)-1-ethyl-2-methylpropyl]phenol hydrochloride (30 g in 100 mL water). The pH of the solution is adjusted to about 7, and then the mixture is stirred at room temperature for about 4 hours. The resulting 3-[3-(dimethylamino)-1-ethyl-2-methylpropyl]phenol resin complex is filtered off and washed with water. The 3-[3-(dimethylamino)-1-ethyl-2-methylpropyl]phenol resin complex is then dried at 50 °C in vacuo.

### Example 4

15.0 g of 3-[3-(dimethylamino)-1-ethyl-2-methylpropyl]phenol resin complex according to Example 1 is blended with a mixture containing 2.0 g of sodium starch glycolate as a disintegrant, 1.0 g of glyceryl behenate as a lubricant, and a proper amount of microcrystalline cellulose. Using a tabletting machine, the mixture can be used to prepare a tablet containing 75 mg of 3-[3-(dimethylamino)-1-ethyl-2-methylpropyl]phenol (calculated as free base).

### Example 5

15.0 g of 3-[3-(dimethylamino)-1-ethyl-2-methylpropyl]phenol resin complex according to Example 2 is blended with 6.0 g of microcrystalline cellulose as a diluent. The mixture is pulverized by compaction and filtered by a 18-mesh sieve to form a granule. To the granule are added 4.0 g of sodium starch glycolate as a disintegrant, 1.0 g of glyceryl behenate as a lubricant, and a proper amount of microcrystalline cellulose such that a total weight may be 35.0 g. Using a tabletting machine, the mixture can be used to prepare a tablet containing 75 mg of 3-[3-(dimethylamino)-1-ethyl-2-methylpropyl]phenol (calculated as free base).

### Example 6

15.0 g of 3-[3-(dimethylamino)-1-ethyl-2-methylpropyl]phenol resin complex according to Example 2 is blended with 1.0 g of glyceryl behenate as a lubricant, 3.0 g of povidone as a binder, and 13.0 g of microcrystalline cellulose as a diluent. The mixture is pulverized by compaction and filtered by a 18-mesh sieve to form a granule. To the granule are added 2.0 g of sodium starch glycolate as a disintegrant, and 1.0 g of glyceryl behenate as a lubricant. Using a capsule filling machine, the final mixture is subjected to moderate pressure to form a slurry which is filled into a capsule.

### Example 7

2.0 g of 3-[3-(dimethylamino)-1-ethyl-2-methylpropyl]phenol is suspended in 34 mL of pure water. Lactic acid is added such that the pH of the solution is adjusted to 4.5. Then 12 g of Amberlite^{™} IRP69 is added and mixed to form a slurry. Subsequently 65 g of malt extract syrup is added to the slurry and mixed therewith. 0.1 g of sorbic acid is dissolved in 2.5 g of propylene glycol, and this solution is mixed with the previously formed slurry. Finally, pure water is added to yield 100 g of liquid preparation.

### Example 8

2.0 g of 3-[3-(dimethylamino)-1-ethyl-2-methylpropyl]phenol is suspended in 34 mL of pure water. Lactic acid is added such that the pH of the solution is adjusted to 4.5. Then 15g of Amberlite^{™} IRP64 is added and mixed well to form a slurry. The pH-value is adjusted to 5.5 by adding 50% w/w NaOH. 0.1 g of sorbic acid is dissolved in 10 g of propylene glycol, and the obtained solution is added to the pH adjusted slurry. Then 1.5 g of colloidal silicon dioxide is added and mixed. Subsequently, 65 g of malt extract syrup is added to the slurry and mixed therewith to yield 100 g of the desired preparation.

### Example 9

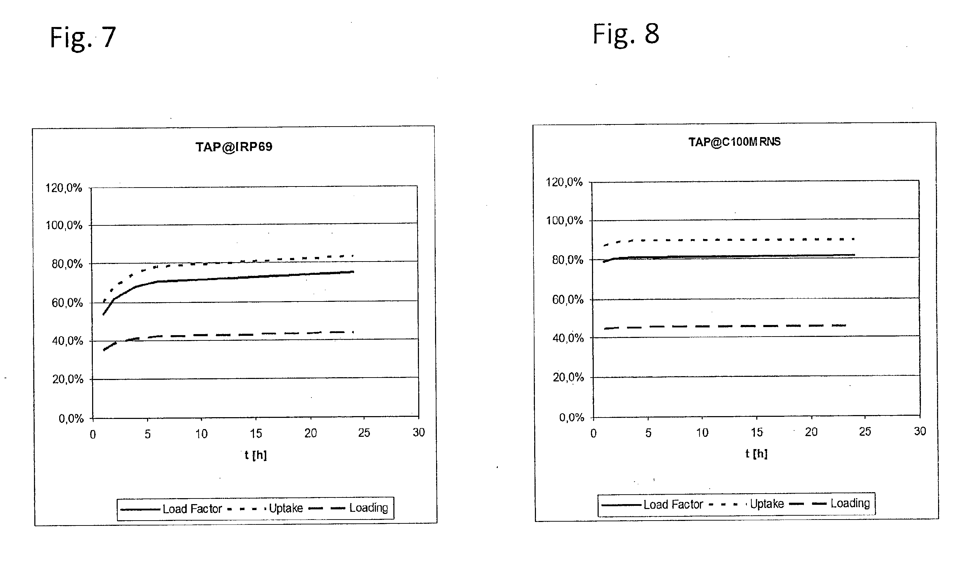

2.5 g of Amberlite^{™} IRP69 were added to a solution of 3-[3-(dimethylamino)-1-ethyl-2-methylpropyl]phenol hydrochloride (2.5 g in 50 mL of water). The mixture was stirred at room temperature for about 4 hours. The resulting 3-[3-(dimethylamino)-1-ethyl-2-methylpropyl]phenol resin complex was filtered off and washed with water. The 3-[3-(dimethylamino)-1-ethyl-2-methylpropyl]phenol resin complex was then dried at 50 °C in vacuo. The initial loading was determined by an in-process control (IPC) via HPLC. The content of 3-[3-(dimethylamino)-1-ethyl-2-methylpropyl]phenol was determined over a period of 1-24 h by release using NaCl solution and analysis using HPLC. Results are shown in Fig. 7. Shortly, after 24h the loading factor was about 75%, the uptake was about 83% and the loading was about 43% (vs. 44% IPC).

### Example 10

2.5 g of Purolite C100MRNS were added to a solution of 3-[3-(dimethylamino)-1-ethyl-2-methylpropyl]phenol hydrochloride (2.5 g in 50 mL of water). The mixture was stirred at room temperature for about 4 hours. The resulting 3-[3-(dimethylamino)-1-ethyl-2-methylpropyl]phenol resin complex was filtered off and washed with water. The 3-[3-(dimethylamino)-1-ethyl-2-methylpropyl]phenol resin complex is then dried at 50 °C in vacuo. The initial loading was determined by an in-process control (IPC) via HPLC. The content of 3-[3-(dimethylamino)-1-ethyl-2-methylpropyl]phenol was determined over a period of 1-24 h by release using NaCl solution and analysis using HPLC. Results are shown in Fig. 8. Contrary to the capacity of Purolite C100MRNS given in the product certificate, the "real" capacity was experimentally determined as 4,3 mmol/g. Shortly, after 24h the loading factor was about 81%, the uptake was about 90% and the loading was about 46% (vs. 46% IPC).

### Example 11

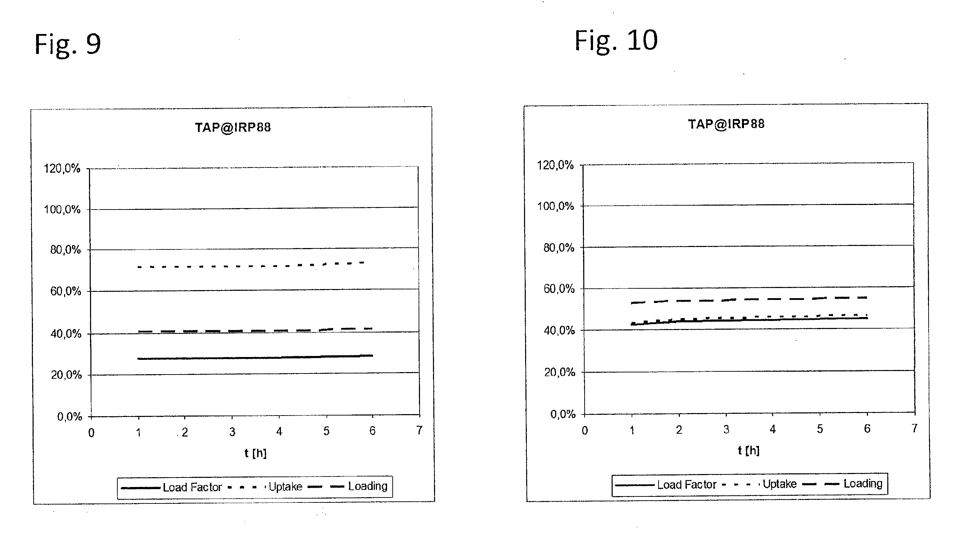

2.5 g of Amberlite^{™} IRP88 were added to a solution of 3-[3-(dimethylamino)-1-ethyl-2-methylpropyl]phenol hydrochloride (2.5 g in 50 mL of water). The mixture was stirred at room temperature for about 4 hours. The resulting 3-[3-(dimethylamino)-1-ethyl-2-methylpropyl]phenol resin complex was filtered off and washed with water. The 3-[3-(dimethylamino)-1-ethyl-2-methylpropyl]phenol resin complex was then dried at 50 °C in vacuo. The initial loading was determined by an in-process control (IPC) via HPLC. The content of 3-[3-(dimethylamino)-1-ethyl-2-methylpropyl]phenol was determined over a period of 1-6 h by release using NaCl solution and analysis using HPLC. Results are shown in Fig. 9. Shortly, after 6h the loading factor was about 28%, the uptake was about 73% and the loading was about 45% (vs. 42% IPC).

### Example 12

1 g of Amberlite^{™} IRP88 were added to a solution of 3-[3-(dimethylamino)-1-ethyl-2-methylpropyl]phenol hydrochloride (2.5 g in 50 mL of water). The mixture was stirred at room temperature for about 4 hours. The resulting 3-[3-(dimethylamino)-1-ethyl-2-methylpropyl]phenol resin complex was filtered off and washed with water. The 3-[3-(dimethylamino)-1-ethyl-2-methylpropyl]phenol resin complex was then dried at 50 °C in vacuo. The initial loading was determined by an in-process control (IPC) via HPLC. The content of 3-[3-(dimethylamino)-1-ethyl-2-methylpropyl]phenol was determined over a period of 1-6 h by release using NaCl solution and analysis using HPLC. Results are shown in Fig. 10. Shortly, after 6h the loading factor was about 45%, the uptake was about 46% and the loading was about 55% (IPC).

### Example 13

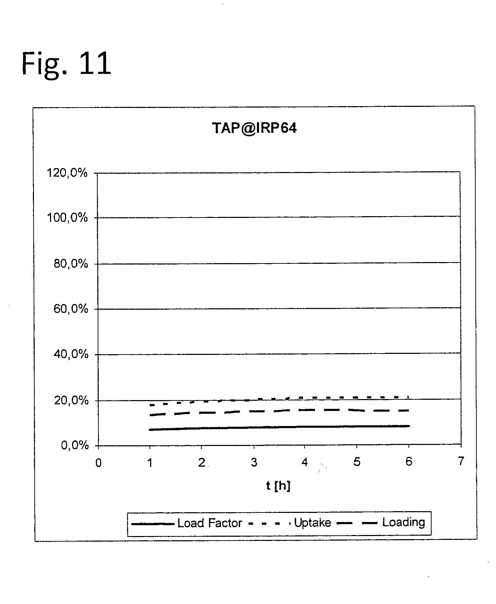

2.5 g of Amberlite^{™} IRP64 were added to a solution of 3-[3-(dimethylamino)-1-ethyl-2-methylpropyl]phenol hydrochloride (2.5 g in 50 mL of water). The mixture was stirred at room temperature for about 4 hours. The resulting 3-[3-(dimethylamino)-1-ethyl-2-methylpropyl]phenol resin complex was filtered off and washed with water. The 3-[3-(dimethylamino)-1-ethyl-2-methylpropyl]phenol resin complex was then dried at 50 °C in vacuo. The initial loading was determined by an in-process control (IPC) via HPLC. The content of 3-[3-(dimethylamino)-1-ethyl-2-methylpropyl]phenol was determined over a period of 1-6 h by release using NaCl solution and analysis using HPLC. Results are shown in Fig. 11. Shortly, after 6h the loading factor was about 8%, the uptake was about 21% and the loading was about 14% (vs. 15% IPC).

### Example 14

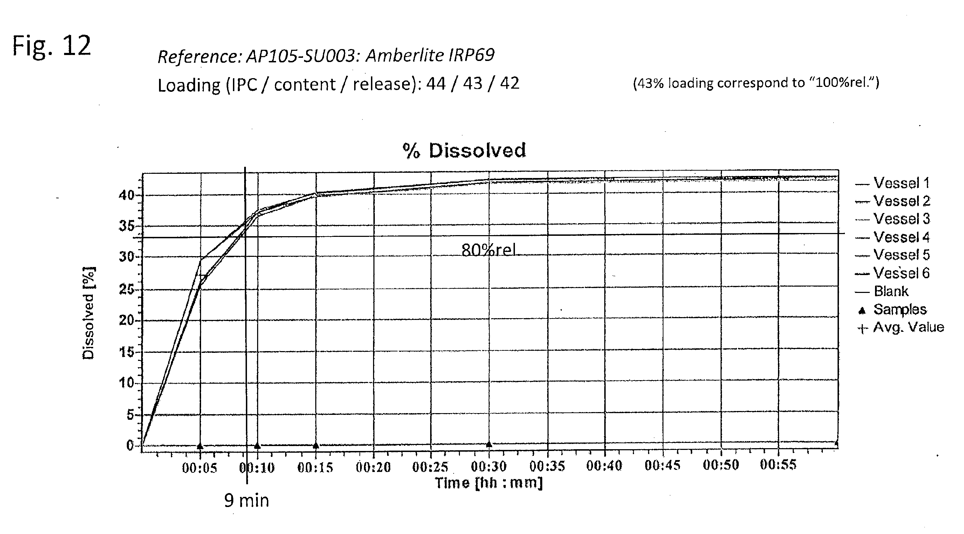

50mg of the resin complex obtained in example 9 were added to 900 ml of a 0.1 M HCl solution in a SOTAX release equipment. The amount of released 3-[3-(dimethylamino)-1-ethyl-2-methylpropyl]phenol in solution was determined in 5 min intervals for 1 h using UV/Vis analysis. Six independent experiments and mean value of said experiments are shown in Fig.12. Shortly, after about 9 min, approximately 80% of 3-[3-(dimethylamino)-1-ethyl-2-methylpropyl]phenol were released.

### Example 15

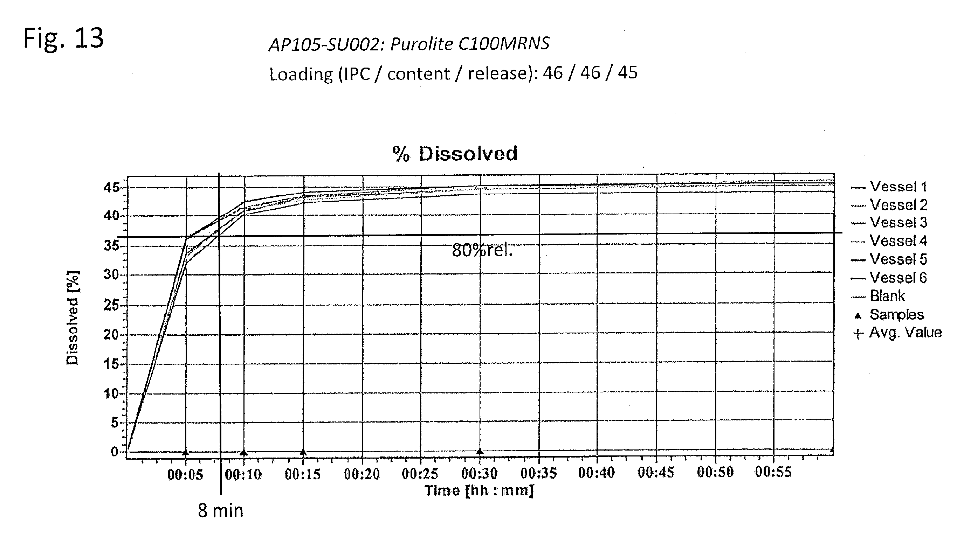

50mg of the resin complex obtained in example 10 were added to 900 ml of a 0.1 M HCl solution in a SOTAX release equipment. The amount of released 3-[3-(dimethylamino)-1-ethyl-2-methylpropyl]phenol in solution was determined in 5 min intervals for 1 h using UV/Vis analysis. Six independent experiments and mean value of said experiments are shown in Fig. 13. Shortly, after about 8 min, approximately 80% of 3-[3-(dimethylamino)-1-ethyl-2-methylpropyl]phenol were released.

### Example 16

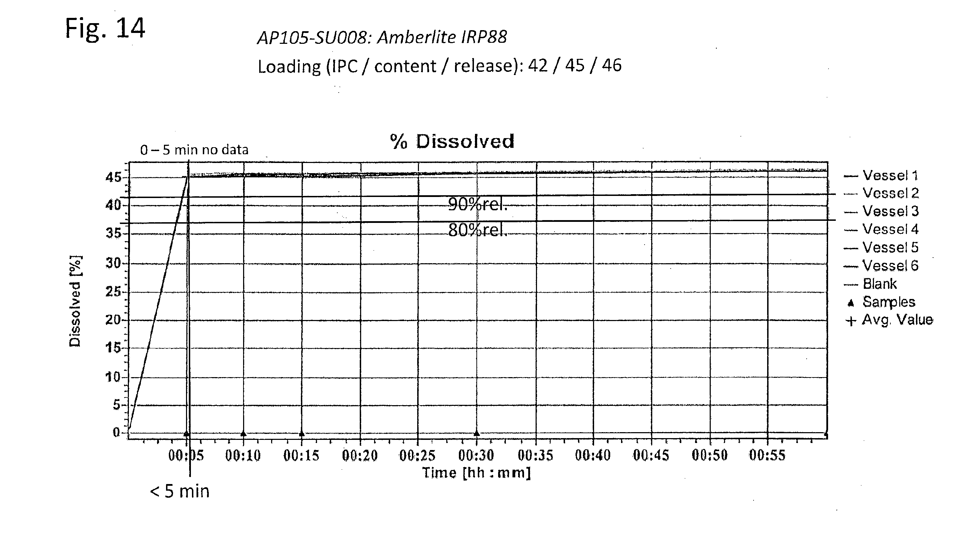

50mg of the resin complex obtained in example 11 were added to 900 ml of a 0.1 M HCl solution in a SOTAX release equipment. The amount of released 3-[3-(dimethylamino)-1-ethyl-2-methylpropyl]phenol in solution was determined in 5 min intervals for 1 h using UV/Vis analysis. Six independent experiments and mean value of said experiments are shown in Fig.14. Shortly, already after 5 min at least 90% of 3-[3-(dimethylamino)-1-ethyl-2-methylpropyl]phenol were released.

### Examples 17-20

Powder X-ray diffraction pattern analysis of 3-[3-(dimethylamino)-1-ethyl-2-methylpropyl]phenol cation exchange resin complex of Amberlite IRP69 (Example 17), Purolite C100MRNS (Example 18), Amberlite^{™} IRP88 (Example 19) or Amberlite^{™} IRP64 (Example 20) was performed using a Bruker AXS D8 ADVANCE diffractometer and the following conditions: Cu-Anode, Ni-Filter; 40kV, 30mA; position sensitive detector (PSD): Lynx Eye; scanning at 0.06° 2-Theta per 0.5 sec; Effective Sample surface: diameter 1.9 mm; rotating; and room temperature. Results are shown in Figs. 1-4 for Examples 17 to 20, respectively. The peak at 2° - 4° 2-Theta was due to Rayleigh-Scattering.

### Reference Example

Powder X-ray diffraction pattern analysis of 3-[3-(dimethylamino)-1-ethyl-2-methylpropyl]phenol hydrochloride crystalline "Form A" using the material and methods as described for examples 17-20. Result is shown in Fig. 5.

## Claims

1. A 3-[3-(dimethylamino)-1-ethyl-2-methylpropyl]phenol resin complex formed by the reaction between a cation exchange resin and 3-[3-(dimethylamino)-1-ethyl-2-methylpropyl]phenol or a pharmaceutically acceptable acid addition salt thereof.

2. The 3-[3-(dimethylamino)-1-ethyl-2-methylpropyl]phenol resin complex according to claim 1, wherein an amorphous form of 3-[(1R,2R)-3-(dimethylamino)-1-ethyl-2-methylpropyl]phenol resin complex is formed by the reaction between a cation exchange resin and 3-[(1R,2R)-3-(dimethylamino)-1-ethyl-2-methylpropyl]phenol or a pharmaceutically acceptable acid addition salt thereof.

3. The 3-[3-(dimethylamino)-1-ethyl-2-methylpropyl]phenol resin complex according to claim 1 or 2, wherein said cation exchange resin is selected from styrene polymers and copolymers of styrene and divinylbenzene, respectively sulphonated or carboxylated, and copolymers of acrylic or methacrylic acid and divinylbenzene, preferably wherein the cation exchange resin is an Amberlite^{™} or a Purolite resin, more preferably wherein the cation exchange resin is Amberlite^{™} IRP69, Amberlite^{™} IRP88, or Purolite C100MRNS.

4. The 3-[3-(dimethylamino)-1-ethyl-2-methylpropyl]phenol resin complex according to any one of the preceding claims, wherein said cation exchange resin is in acid form or in the form of a salt with an alkali metal ion as counter ion.

5. The 3-[3-(dimethylamino)-1-ethyl-2-methylpropyl]phenol resin complex according to any one of the preceding claims, wherein said cation exchange resin is a sulfonated styrene divinylbenzene resin, preferably in salt form.

6. The 3-[3-(dimethylamino)-1-ethyl-2-methylpropyl]phenol resin complex according to any of the preceding claims containing from 1% up to 100% of 3-[3-(dimethylamino)-1-ethyl-2-methylpropyl]phenol, expressed as the ratio of the number of actually 3-[3-(dimethylamino)-1-ethyl-2-methylpropyl]phenol-occupied cationic exchange sites to the total number theoretically available exchange sites of the resin.

7. The 3-[3-(dimethylamino)-1-ethyl-2-methylpropyl]phenol resin complex according to claim 6 containing 30% to 98%, preferably 35% to 98% , more preferably 40% to 98%, and in particular 45% to 95% of 3-[3-(dimethylamino)-1-ethyl-2-methylpropyl]phenol, expressed as the ratio of the number of actually 3-[3-(dimethylamino)-1-ethyl-2-methylpropyl]phenol-occupied cationic exchange sites to the total number theoretically available exchange sites of the resin.

8. A process for producing the 3-[3-(dimethylamino)-1-ethyl-2-methylpropyl]phenol resin complex according to any of claims 1 to 7, comprising mixing 3-[3-(dimethylamino)-1-ethyl-2-methylpropyl]phenol, as free base or as acid addition salt, and a cation exchange resin in an aqueous medium to obtain a 3-[3-(dimethylamino)-1-ethyl-2-methylpropyl]phenol resin complex, then optionally washing and drying the obtained 3-[3-(dimethylamino)-1-ethyl-2-methylpropyl]phenol resin complex.

9. The process according to claim 8, wherein the produced 3-[3-(dimethylamino)-1-ethyl-2-methylpropyl]phenol resin complex is defined as in any of the preceeding claims 2 to 7.

10. The process according to claim 8 or 9, wherein 3-[3-(dimethylamino)-1-ethyl-2-methylpropyl]phenol or an acid addition salt thereof and the cation exchange resin are mixed in ranges of weight ratios 3-[3-(dimethylamino)-1-ethyl-2-methylpropyl]phenol to resin of 1:0.1 to 1:15, preferably 1:0.5 to 1:12, and more preferably 1:0.9 to 1:6, calculated on the basis of 3-[3-(dimethylamino)-1-ethyl-2-methylpropyl]phenol free base.

11. A pharmaceutical composition comprising the 3-[3-(dimethylamino)-1-ethyl-2-methylpropyl]phenol resin complex according to claim 1, optionally in combination with a further pharmaceutically acceptable excipient, additive or vehicle.

12. The pharmaceutical composition according to claim 11, wherein the 3-[3-(dimethylamino)-1-ethyl-2-methylpropyl]phenol resin complex is defined by any of the preceeding claims 2 to 7.

13. The pharmaceutical composition according to claim 11 or 12, comprising the 3-[3-(dimethylamino)-1-ethyl-2-methylpropyl]phenol resin complex in an amount of about 0.1 to 80wt.-%, preferably between 0.5 to 60wt.-%, and more preferably between 1 to 30wt.-% relative to the total weight of the pharmaceutical composition.

14. The pharmaceutical composition according to anyone of claims 11 to 13, comprising excipients, additives or vehicles selected from the group consisting of solvents or dispersing media, diluents, binders, disintegrants, stabilizers, fillers, buffers, coloring agents, sweetening agents, flavoring agents, preservatives, lubricants, suspending agents, and mixtures thereof.

15. The 3-[3-(dimethylamino)-1-ethyl-2-methylpropyl]phenol resin complex according to claims 1 to 7, wherein said 3-[3-(dimethylamino)-1-ethyl-2-methylpropyl]phenol or a pharmaceutically acceptable acid addition salt thereof contains or consists of 3-[(1R,2R)-3-(dimethylamino)-1-ethyl-2-methylpropyl]phenol or a pharmaceutically acceptable acid addition salt thereof or 3-[(1S,2S)-3-(dimethylamino)-1-ethyl-2-methylpropyl]phenol or a pharmaceutically acceptable acid addition salt thereof or 3-[(1R,2S)-3-(dimethylamino)-1-ethyl-2-methylpropyl]phenol or a pharmaceutically acceptable acid addition salt thereof or
3-[(1S,2R)-3-(dimethylamino)-1-ethyl-2-methylpropyl]phenol or a pharmaceutically acceptable acid addition salt thereof,
preferably of 3-[(1R,2R)-3-(dimethylamino)-1-ethyl-2-methylpropyl]phenol or a pharmaceutically acceptable acid addition salt thereof.
